# EUROPÄISCHE PATENTSCHRIFT

(11) **EP 0 380 712 B1**
(45) Veröffentlichungstag und Bekanntmachung des Hinweises auf die Patenterteilung: **04.01.1995**
(21) Anmeldenummer: 89101437.5
(22) Anmeldetag: 27.01.1989
(51) Int. Cl.: C07C 227/22, C07C 229/42

(54) **Verfahren zur Herstellung von 2,6-Dichlordiphenylaminessigsäurederivaten**
Process for the preparation of 2,6-dichlorodiphenylaminoacetic-acid derivatives
Procédé de préparation de dérivés de l'acide 2,6-dichlorodiphénylaminoacétique

(43) Veröffentlichungstag der Anmeldung: 08.08.1990
(73) Patentinhaber: HEUMANN PHARMA GMBH & CO, D-90478 Nürnberg (DE)
(72) Erfinder: Grafe, Ingomar, Dr. Dipl.Chem., D-8500 Nürnberg 30 (DE); Schickaneder, Helmut, Dr. Dipl.Chem., D-8501 Eckental (DE); Ahrens, Kurt-Henning, Dr., D-8500 Nürnberg (DE)
(74) Vertreter: Kraus, Walter, Dr.

(56) Entgegenhaltungen:
- US-A- 3 558 690

## Beschreibung

2,6-Dichlorsubstituierte Diphenylaminessigsäurederivate spielen eine herausragende Rolle bei der Therapie rheumatischer Erkrankungen, bei der sich vor allem Diclofenac Natrium der Formel I
(R = Natriumatom) 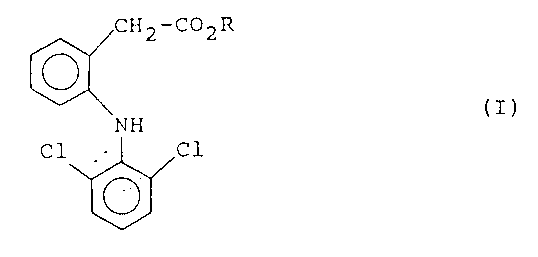
bewährt hat. Diese Verbindung und ihre arzneiliche Verwendung ist bereits aus der DE-A-15 43 639 bekannt. Im allgemeinen verläuft die Herstellung dieser Verbindung über ein Zwischenprodukt der Formel V 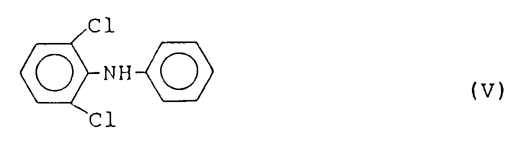
Zur Herstellung dieses Zwischenprodukts, das den zentralen Baustein des oben genannten Wirkstoffs darstellt, sind bereits verschiedene Herstellungsverfahren bekannt, deren Nachteile einerseits in der notwendigen Anwendung von langen Reaktionszeiten und von hohen Temperaturen und andererseits in der Tatsache, daß nur mäßige bis schlechte Ausbeuten erzielt werden, zu sehen sind.

So ist für das Diphenylaminderivat der Formel V bereits ein Verfahren bekannt, das von 2,6-Dichloracetanilid und Brombenzol ausgeht und lange Reaktionszeiten, z.B. 96 h, erfordert (vgl. D.C. Atkins et al., J.Med.Chem. 26, 1361 (1983) ; CH-PS-473 770 und US-PS-3 536 758). Bei einem weiteren Verfahren geht man von Jodbenzol aus. Ausbeuteangaben dieses Verfahrens fehlen allerdings (vgl. JP-OS-57 167 947 und C.A. 98, 53 379 K (1983)).

Auch bei einem neueren Verfahren (vgl. J. S. Kaltenbronn, R.A. Scherer, Arzneim. Forsch. 33, 62 (1983)) wird die angestrebte Substanz nur mit schlechter Ausbeute erhalten. Bei dem Verfahren der DE-OS-15 43 639 beträgt die Ausbeute, wie dort angegeben, nur 43% der Theorie. Nach den Angaben der oben genannten Schriften soll das Zwischenprodukt der Formel V als gelbes Öl anfallen. Tatsächlich handelt es sich aber bei dieser Substanz um einen farblosen Feststoff (Fp. 47,5 - 48°C).

In der JP-OS-58 144 350 wird die Chapman-Umlagerung des N-Phenyl-o-(2,6-dichlorphenyl)benzimidoesters bei 275°C als Alternative zur Darstellung der Verbindung V beschrieben.

Zur Herstellung von Diclofenac-Natrium sind auch schon direkte Verfahren beschrieben worden, die sich jedoch nicht reproduzieren lassen. Bei einem bekannten Verfahren dieser Art wird 2-Chlorphenylessigsäure mit 2,6-Dichloranilin umgesetzt (JP-OS-81 158 744, C.A. 97, 23 467 z (1980)). Das angestrebte Produkt wird nach 12 h in DMF bei 150°C allerdings nur mit einer Ausbeute von 37% erhalten. Auch Verfahren, bei denen 2-Bromphenylessigsäure und 2,6-Dichlorphenylnatriumamid als Ausgangsprodukte verwendet werden, liefern die angestrebte Substanz nur in geringen Ausbeuten (22% in HMPT; vgl. JP-OS-77 00 240, C.A. 87 39 078 c (1977)).

Andere Verfahren arbeiten mit molaren Mengen an Jodverbindungen (vgl. JP-OS-66 550, C.A. 93, 167 946 v (1980) und DE-OS-26 32 198).

Ein Verfahren, bei dem die angestrebte Verbindung der Formel I direkt aus der Verbindung der Formel V erhalten wird, ist in der CH-PS-473 770 und der DE-OS-15 43 639 bekannt. Dieses Verfahren baut sich auf der bekannten Stollé-Reaktion (Ber. dtsch. Chem. Ges. 47, 2120 (1914)) auf und wird in einer Aluminiumchloridschmelze durchgeführt.

Aufgabe der Erfindung ist es, ein Verfahren zur Herstellung der Verbindung der Formel I zur Verfügung zu stellen, das in technisch einfacher und realisierbarer Weise die gewünschte Substanz in sehr guter Reinheit und guter Ausbeute aus gut zugänglichen Rohstoffen liefert, ohne daß die extremen Reaktionsbedingungen der bekannten Verfahren angewendet werden müssen, um das sterisch anspruchsvolle Molekül der Formel V herzustellen.

Diese Aufgabe wird durch die Erfindung gelöst. Gegenstand der Erfindung ist daher ein Verfahren zur Herstellung von 2,6-Dichlordiphenylaminderivat der allgemeinen Formel I 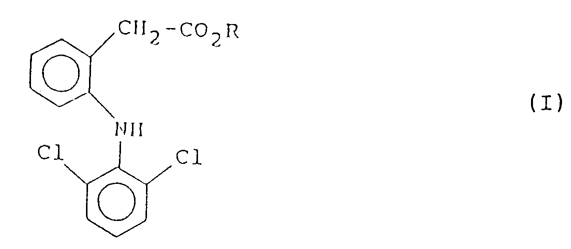
worin R ein Wasserstoffatom, eine C₁- bis C₄-Alkylgruppe oder ein Alkaliatom bedeutet, das dadurch gekennzeichnet ist, daß man ein 2,6-Dichlorphenoxyessigsäurederivat der Formel II 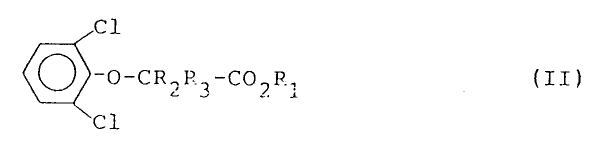
worin R₁ ein Wasserstoffatom oder eine C₁ - bis C₆-Alkylgruppe bedeutet und R₂, R₃ jeweils für ein Wasserstoffatom oder eine C₁- bis C₄-Alkylgruppe stehen, mit Anilin in einer Eintopfreaktion zuerst zu der Verbindung der Formel III 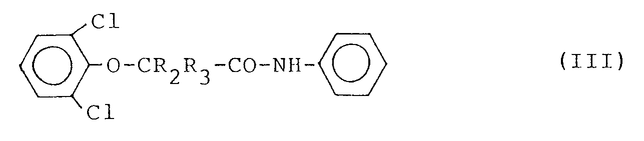
worin R₂ und R₃ die oben angegebenen Bedeutungen haben, umsetzt, dann in situ zu der Verbindung der Formel IV 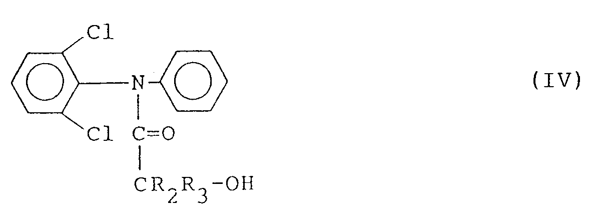
worin R₂ und R₃ die oben angegebenen Bedeutungen haben, umlagert, die erhaltene Verbindung der Formel IV in Gegenwart katalytischer bis äquimolarer Mengen eines Alkalialkoholats zu der Verbindung der Formel V 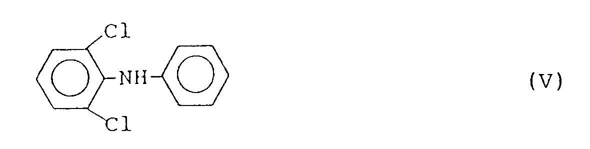
aminolysiert, die Verbindung der Formel V nach Isolierung durch Umsetzung mit chloracetylchlorid in die Verbindung der Formel VI 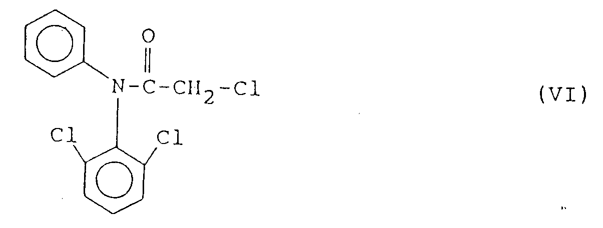
überführt, die Verbindung der Formel VI zu der Verbindung der Formel VII 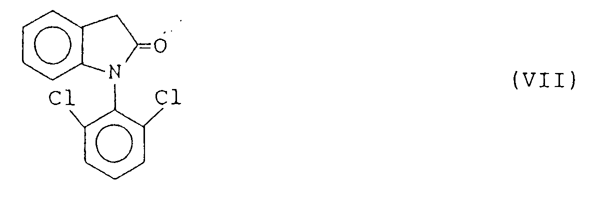
cyclisiert und die erhaltene Verbindung der Formel VII durch alkalische Hydrolyse in die Verbindung der Formel I überführt.

In der ersten Stufe des erfindungsgemäßen Verfahrens wird ein 2,6-Dichlorphenoxyester der Formel II 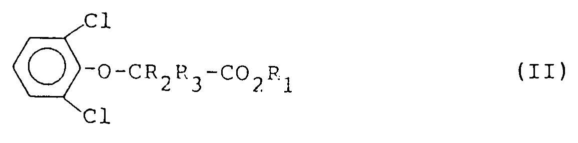
worin R₁ ein Wasserstoffatom oder eine C₁- bis C₆-Alkylgruppe bedeutet und R₂ und R₃ jeweils Wasserstoff oder eine C₁- bis C₄-Alkylgruppe bedeuten, mit Anilin in Gegenwart katalytischer Mengen eines Alkalialkoholats wie Natriummethanolat in einem Alkohol, beispielsweise Methanol, Ethanol, n-Propanol, i-Propanol, n-Butanol oder i-Butanol, bei Temperaturen unter 120°C, beispielsweise bei 90°C vorzugsweise 100°C umgesetzt.

Nach einer bevorzugten Ausführungsfrom der Erfindung wird die Ausgangsverbindung der Formel II aus 3,5-Dichlorhydroxybenzoesäure der Formel VIII 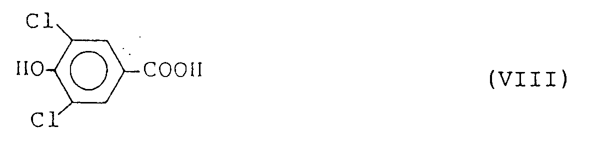
hergestellt. Hierzu wird die Verbindung der Formel VIII in einer Eintopfreaktion zunächst einer Decarboxylierungsreaktion in Dimethylformamid oder Dimethylacetamid in Gegenwart von katalytischen Mengen (z.B. 1/10mol %) von Collidin und bei Temperaturen unter 160°C, beispielsweise 156°C, unterworfen und anschließend sofort mit einem Chloressigsäurederivat der Formel IX 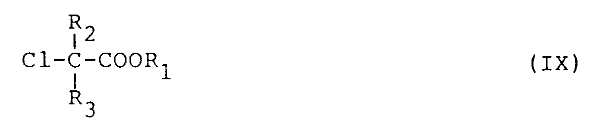
umgesetzt. Dabei kann vorzugweise Kaliumcarbonat als Base verwendet werden, ohne daß eine Verseifung des Esters erfolgt.

Die Zwischenverbindung der Formel III läßt sich auch direkt aus 2,6-Dichlorphenol und Chloracetanilid durch basenkatalysierte Reaktion herstellen; dieses Verfahren eignet sich jedoch wegen der toxischen Nebenwirkung des Chloracetanilids nicht zur Herstellung größer Mengen der Verbindung III.

In der zweiten Stufe des erfindungsgemäßen Verfahrens wird die erfindungsgemäß erhaltene Zwischneverbindung der Formel III ohne Isolierung direkt in situ, bei Reaktionstemperaturen von 90°C bis 100°C, beispielsweise 95°C bis 100°C zu der Verbindung der Formel IV 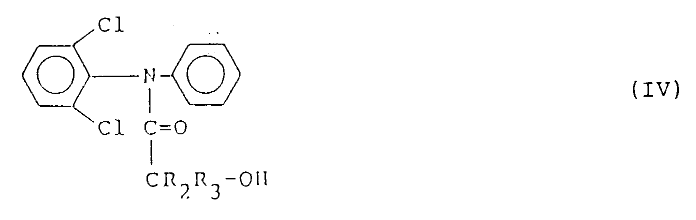
umgelagert. Die Umlagerung wird durch das im Reaktionsmedium vorhandene Alkalialkoholat bewirkt. Wie oben bereits ausgeführt wurde, besteht das Reaktionsmedium aus einem Alkohol, z.B. Methanol, Ethanol, n-Propanol, i-Propanol, n-Butanol oder i-Butanol.

In der dritten Stufe des erfindungsgemäßen Verfahrens wird - ebenfalls ohne Isolierung - die durch Umlagerung erhaltene Verbindung der Formel IV zu der Verbindung der Formel V 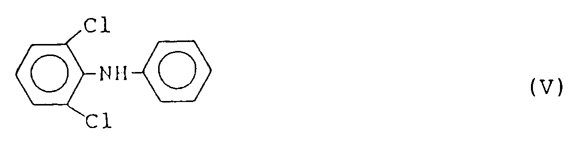
aminolysiert, wobei die Aminolyse durch das im Reaktionsgemisch enthaltene. Alkalialkoholat bewirkt wird. Die Reaktionstemperaturen sind wie in der dritten Stufe des erfindungsgemäßen Verfahrens. Das erhaltene 2,6-Dichlorphenylamin der Formel V wird durch übliche Maßnahmen isoliert und ggf. gereinigt. Die genannte Verbindung fällt als Feststoff an.

In der vierten Stufe des erfindungsgemäßen Verfahrens wird die Verbindung der Formel V durch Umsetzung mit Chloracetylchlorid in das Derivat der Formel VI 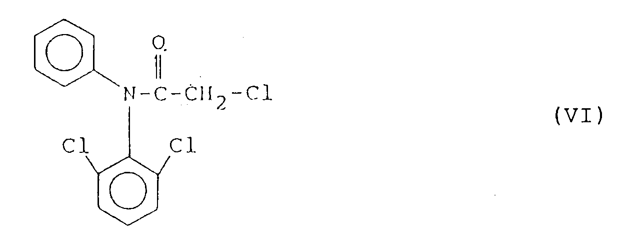
überführt. Erfindungsgemäß erfolgt die Chloracetylierung vorzugsweise als Aminolyse in einem inerten Lösungsmittel, vorzugsweise Xylol oder Chlorbenzol, und bei Temperaturen von 90°C bis 120°C, vorzugsweise 110°C bis 120°C. Nach einer bevorzugten Ausführungsform der Erfindung wird diese Stufe in Anwesenheit katalytischer Mengen, z.B. 1/20 mol % bis 1/10 mol %, 4-Dimethylaminopyridin durchgeführt. Die Ausbeuten an der angestrebten Verbindung der Formel VI sind fast quantitativ.

In der fünften Stufe des erfindungsgemäßen Verfahrens erfolgt eine intramolekulare Cyclisierung der Verbindung der Formel VI zum Indolinon der Formel VII 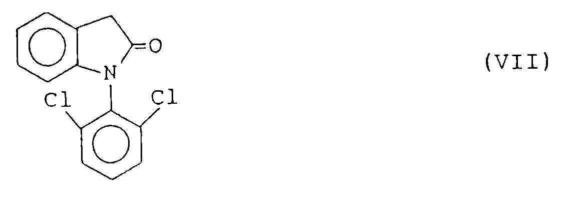
Diese Stufe des erfindungsgemäßen Verfahrens erfolgt in einem inerten Lösungsmittel, vorzugsweise o-Dichlorbenzol, und erhöhten Temperaturen, vorzugsweise 140°C bis 160°C. Dabei werden Ausbeuten von über 90% erreicht.

In der letzten Stufe des erfindungsgemäßen Verfahrens wird die Verbindung der Formel VII durch alkalische Hydrolyse vorzugsweise mit Natronlauge in einem Alkohol wie oben beschrieben, insbesondere n-Butanol, in das Natriumsalz der angestrebten Verbindung der Formel I umgewandelt. Die Reaktionstemperatur in dieser Stufe ist 90°C bis 110°C, vorzugsweise 100°C bis 110°C.

Die so erhaltene Verbindung der Formel I kann durch übliche Maßnahmen, bespielsweise durch Extraktion, aus dem Reaktionsgemisch isoliert werden und durch übliche Verfahrensweisen, beispielsweise Kristallisation, gereinigt werden.

Als wesentliches Merkmal der vorliegenden Erfindung ist anzusehen, daß die erste, die zweite und die dritte Stufe des Verfahrens als Eintopfverfahren durchgeführt wird, ohne daß dei Zwischneverbindungen III und IV isoliert werden. Auf diese Weise werden einerseits eine einfache Verfahrensführung und andererseits relativ hohe Gesamtausbeuten, z.B. 80% bis 90% d. Th., erhalten.

Die Erfindung wird an den folgenden Beispielen erläutert.

### Beispiel 1

### Darstellung von 2,6-dichlorphenoxyessigsäureethylester

In 0,5 l DMF trägt man bei ca. 90°C 675 g 3,5-Dichlor-4-hydroxybenzoesäure ein, gibt 75 ml Collidin zu und heizt bis 120°C, wobei die Decarboxylierung einsetzt.

Die Sumpftemperatur wird langsam auf 155°C gesteigert und gehalten bis kein CO₂ mehr entweicht.

Der Sumpf wird bei 50°C mit 240 g Pottasche, 0,6 l Essigester und 0,35 l Chlroessigsäureethylester versetzt. Man wartet bei 70°C ide Umsetzung ab, die unter Freisetzung von CO₂ verläuft, und kocht noch 2 h unter Rückfluß. Nach Zusatz von 1,7 l Wasser führt man bei 20°C eine Phasentrennung durch und fraktioniert die organische Phase.
- Kp.: 0,1 = 100 bis 130°C
- Ausbeute:: 617 g = 83% d. Th.

### Beispiel 2

### Darstellung von 2,6-Dichlordiphenylamin

Ein Gemisch aus 996 g 2,6-Dichlorphenoxyessigethylester, 500 ml Anilin und 400 ml n-Butanol wird in einer Destillationsapparatur auf 100°C vorgeheizt.

In 30 min läßt man 400 ml 5,5 n Natriummethylat zufließen, wobei ein Destillatfluß durch die Exothermie der Umsetzung aufrechterhalten wird. Nach der Zugabe wird noch 15 min bei 100°C unter weiterer Destillatabnahme gerührt.

Dann gibt man 1 l Wasser zu und destilliert Lösungsmittel bis zur Sumpftemperatur von 90°C ab. Bei 60°C erfolgt Phasentrennung. Das Lösungsmittel wird imn Vakuum abgezogen und das Produkt aus 1 l 90% i-Propanol kristallisiert. Man saugt bei 0°C ab und wäscht mit dil. i-Propanol nach.
- Ausbeute:: 727 g = 76,5% d. Th.
bei Ausbeutung der Mutterlauge 82% d. Th.
- DSCₘₐₓ =: 54°C bei 2°C/min Heizrate

### Beispiel 3

### Darstellung von N-(2,6-Dichlorphenyl)indolinon(2)

In 900 ml o-Dichlorbenzol werden 476 g 2,6-Dichlordiphenylamin vorgelegt und bei 150°C mit 271 g Chloracetylchlorid in 1,5 h versetzt. Das Gemisch wird noch 2 h bie 150°C gerührt, bis keine Gase mehr entweichen.

Bei 60°C werden 533 g Aluminiumchlorid zugegeben und das Gemisch langsam bis 160°C geheizt, wobei ab 100°C Chlorwasserstoff entweicht. Nach 1 h bei 110°C kühlt man auf 60°C und hydrolysiert bei maximal 80°C mit 1,5 l Eiswasser.

Nach Phasentrennung wäscht man nochmals mit Wasser und zieht das Lösungsmittel im Vakuum ab. Das Produkt wird aus 1 l i-Propanol kristallisiert.
- Ausbeute:: 512 g = 92% d. Th.
- DSCₘₐₓ =: 121°C bei 2°C/min Heizrate

### Beispiel 4

### Darstellung von Diclofenac Natrium

1 Teil des Indolinons aus Beispiel 3 wird in 2 Teilen n-Butanol und der äquivalenten Menge Natronlauge unter azeotroper Wasserentfernung gekocht, wobei 0,2 Teile Toluol zugesetzt werden.

Wenn kein Wasser mehr abgeschieden wird und das Salz gefallen ist, gibt man 2 TL i-Propanol zu und saugt bei 10°C ab.

Dieses Produkt wird aus Wasser umkristallisiert und bei 100°C getrocknet.
- Ausbeute:: 88,6% d. Th.
- DSCₘₐₓ =: 106°C (bei Hydrat)
- DSCₘᵢₙ =: 268 bis 270°C bei 2°C/min Heizrate

## Patentansprüche

1. Verfahren zur Herstellung von 2,6-Dichlordiphenylaminderivaten der allgemeinen Formel I 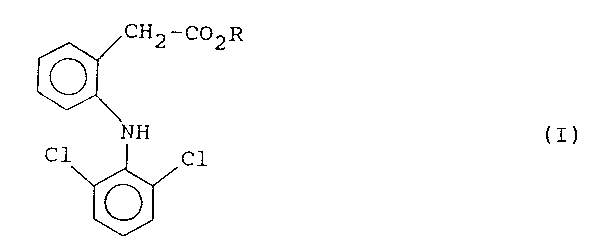 worin R ein Wasserstoffatom, eine C₁- bis C₄-Alkylgruppe oder ein Alkaliatom bedeutet, dadurch **gekennzeichnet,** daß man ein 2,6-Dichlorphenoxyessigsäurederivat der Formel II 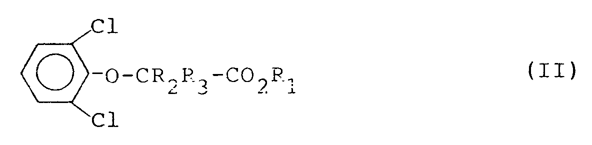 worin R₁ ein Wasserstoffatom oder eine C₁- bis C₆-Alkylgruppe bedeutet und R₂, R₃ jeweils für ein Wasserstoffatom oder eine C₁- bis C₄-Alkylgruppe stehen,
mit Anilin in einer Eintopfreaktion zuerst zu der Verbindung der Formel III 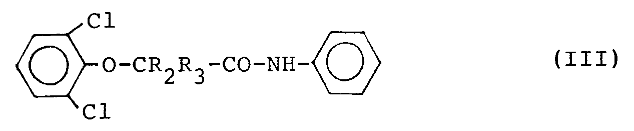 worin R₂ und R₃ die oben angegebenen Bedeutungen haben, umsetzt, dann in situ zu der Verbindung der Formel IV 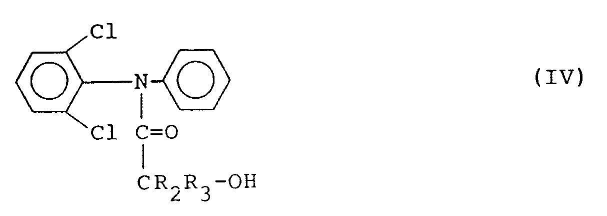 worin R₂ und R₃ die oben angegebenen Bedeutungen haben, umlagert, die erhaltene Verbindung der Formel IV in Gegenwart katalytischer bis äquimolarer Mengen eines Alkalialkoholats zu der Verbindung der Formel V 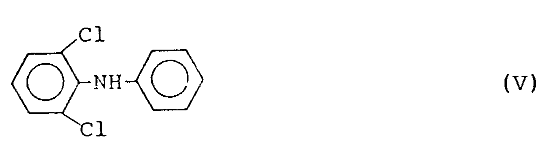 aminolysiert, die Verbindung der Formel V nach Isolierung durch Umsetzung mit Chloracetylchlorid in die Verbindung der Formel VI 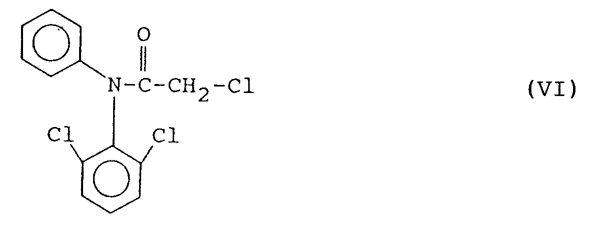 überführt, die Verbindung der Formel VI zu der Verbindung der Formel VII 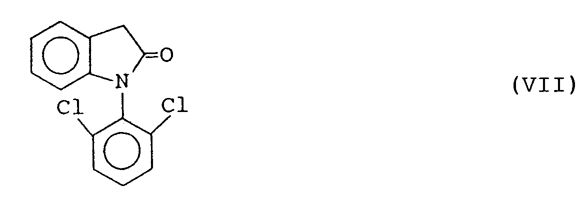 cyclisiert und die erhaltene Verbindung der Formel VII durch alkalische Hydrolyse in die Verbindung der Formel I überführt.

2. Verfahren nach Anspruch 1, dadurch **gekennzeichnet,** daß man die Verbindung der Formel II aus 3,5-Dichlor-4-hydroxybenzoesäure dem Formel VIII 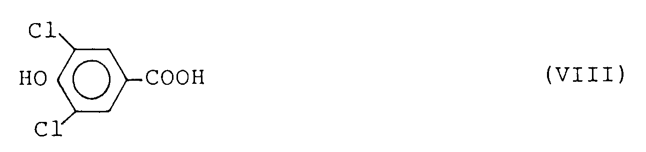 durch Decarboxylierung in Dimethylformanid oder Dimethylacetamid in Gegenwart von katalytischen Mengen Collidin und Umsetzung mit einem Chloressigsäurederivat der Formel IX 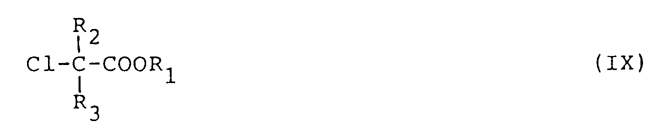 in der R₁, R₂ und R₃ wie in Anspruch 1 definiert sind, in einer Eintopfreaktion herstellt.

3. Verfahren nach Anspruch 1, dadurch **gekennzeichnet,** daß man die Herstellung der Verbindung der Formel V in einem Alkohol bei Temperaturen unter 120°C vornimmt.

4. Verfahren nach Anspruch 1, dadurch **gekennzeichnet,** daß man die Umsetzung der Verbindung der Formel V mit Chloracetylchlorid in Form einer Aminolyse durchführt und die erhaltene Verbindung der Formel VI in o-Dichlorbenzol zu der Verbindung der Formel VII cyclisiert und diese mit Alkalilauge in einem protischen Lösungsmittel in die Verbindung der Formel I überführt.

## Claims

1. Process for the preparation of 2,6-dichlorodiphenylamino derivatives of the general formula I 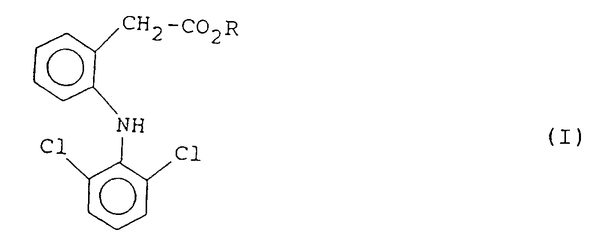 wherein R signifies a hydrogen atom, a C₁- to C₄-alkyl group or an alkali atom, characterised in that a 2,6-dichlorophenoxyacetic acid derivative of the formula II 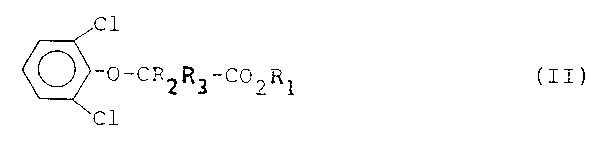 wherein R₁ signifies a hydrogen atom or a C₁- to C₆-alkyl group and R₂, R₃ each represent a hydrogen atom or a C₁- to C₄-alkyl group, is first of all reacted with aniline in a one-pot reaction to the compound of the formula III 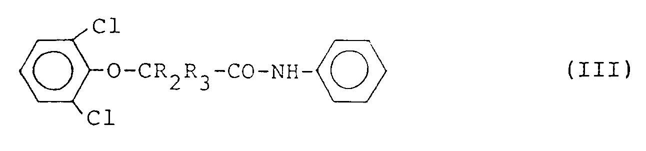 wherein R₂ and R₃ have the meanings given above, then rearranged in situ to the compound of the formula IV 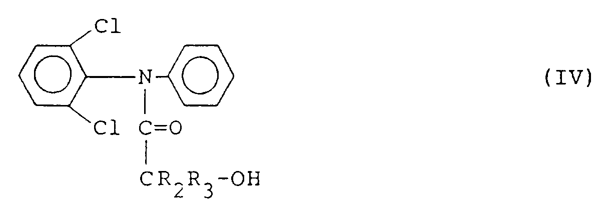 wherein R₂ and R₃ have the meanings given above, the compound of the formula IV obtained is aminolysed in the presence of catalytic to equimolar quantities of an alkali alcoholate to the compound of the formula V, 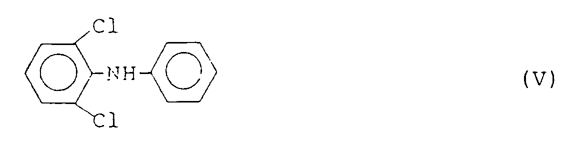 the compound of the formula V is isolated and then converted by reaction with chloroacetyl chloride into the compound of the formula VI, 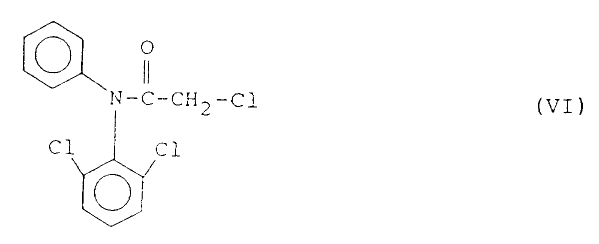 the compound of the formula VI is cyclised to the compound of the formula VII 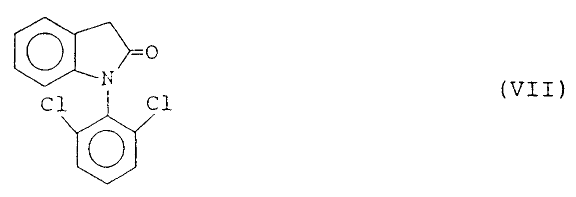 and the compound of the formula VII obtained is converted by alkaline hydrolysis into the compound of the formula I.

2. Process according to claim 1, characterised in that the compound of the formula II is prepared in a one-pot reaction from 3,5-dichloro-4-hydroxybenzoic acid of the formula VIII 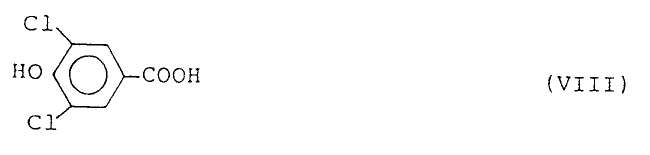 by decarboxylation in dimethylformamide or dimethylacetamide in the presence of catalytic quantities of collidine and reaction with a chloroacetic acid derivative of the formula IX 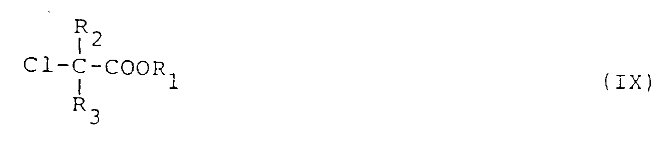 wherein R₁, R₂ and R₃ are defined as in claim 1.

3. Process according to claim 1, characterised in that the preparation of the compound of the formula V is performed in an alcohol at temperatures of below 120°C.

4. Process according to claim 1, characterised in that the reaction of the compound of the formula V with chloroacetyl chloride is carried out in the form of an aminolysis and the compound of the formula VI obtained is cyclised in o-dichlorobenzene to form the compound of the formula VII and the latter is converted into the compound of the formula I by means of lye in a protonic solvent.

## Revendications

1. Procédé de préparation de dérivés de 2,6-dichlorophénylamine de formule générale I 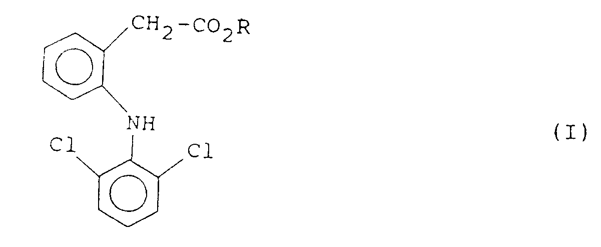 dans laquelle R est un atome d'hydrogène, un groupement alkyle en C₁-C₄ ou un atome d'alcali, caractérisé en ce qu'on transforme tout d'abord un dérivé d'acide 2,6-dichloro-phénoxyacétique de formule II 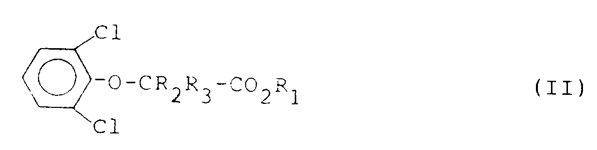 dans laquelle R₁ est un atome d'hydrogène ou un groupement alkyle en C₁-C₆ et R₂, R₃ représentent respectivement un atome d'hydrogène ou un groupement alkyle en C₁-C₄,
avec de l'aniline, au cours d'une réaction dans un seul récipient, en composé de formule III 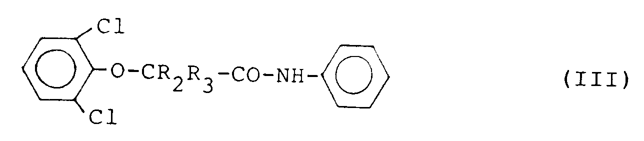 dans laquelle R₂ et R₃ ont les significations susmentionnées, puis qu'on le transpose in situ en composé de formule IV 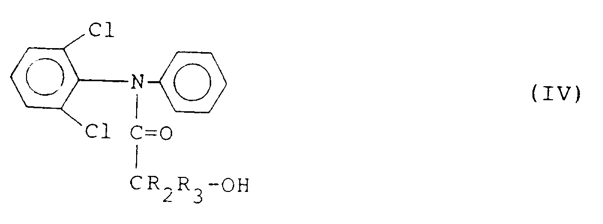 dans laquelle R₂ et R₃ ont les significations susmentionnées, qu'on aminolyse le composé de formule IV obtenu en présence de quantités catalytiques à équimolaires d'un alcoolate d'alcali, en composé de formule V 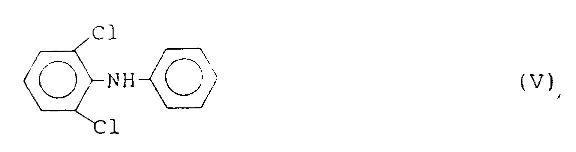 qu'on transforme le composé de formule V après isolement par réaction avec du chlorure de chloracétyle en composé de formule VI 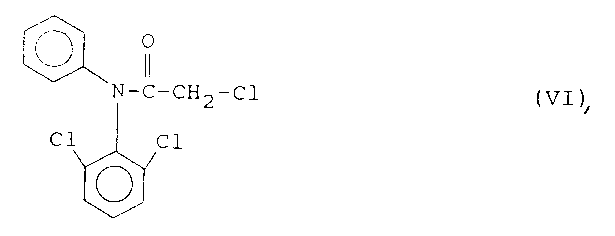 qu'on cyclise le composé de formule VI en composé de formule VII 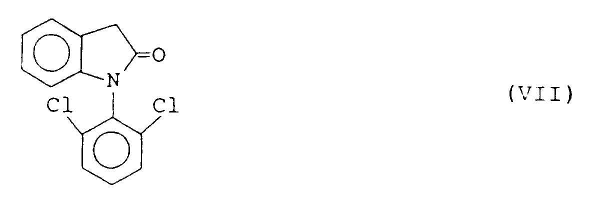 et qu'on transforme le composé de formule VII obtenu par hydrolyse alcaline en composé de formule I.

2. Procédé selon la revendication 1, caractérisé en ce qu'on prépare le composé de formule II à partir d'acide 3,5-dichloro-4-hydroxybenzoïque de formule VIII 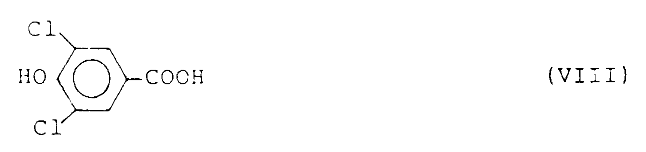 par décarboxylation dans du diméthylformamide ou du diméthylacétamide en présence de quantités catalytiques de collidine et réaction avec un dérivé d'acide chloracétique de formule IX 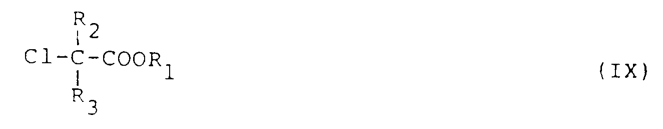 dans laquelle R₁, R₂ et R₃ sont tels que définis à la revendication 1, au cours d'une réaction dans un seul récipient.

3. Procédé selon la revendication 1, caractérisé en ce qu'on procède à la préparation du composé de formule V dans un alcool à des températures inférieures à 120°C.

4. Procédé selon la revendication 1, caractérisé en ce qu'on effectue la réaction du composé de formule V avec du chlorure de chloracétyle sous forme d'une aminolyse et qu'on cyclise le composé de formule VI obtenu dans du o-dichlorobenzène en composé de formule VII et qu'on transforme ce dernier en composé de formule I à l'aide d'un hydroxyde d'alcali en solution dans un solvant protique.
